# EUROPEAN PATENT APPLICATION

(11) **EP 3 473 724 A1**
(43) Date of publication of application: **24.04.2019**
(21) Application number: 17382699.1
(22) Date of filing: 19.10.2017
(51) Int. Cl.: C12P 5/02, C12M 1/00, C12M 1/34

(54) **METHOD FOR OBTAINING METHANE ENRICHED BIOGAS AND AN INSTALLATION FOR CARRYING OUT SAID METHOD**

(71) Applicant: FCC Aqualia, S.A., 28050 Madrid (ES)
(72) Inventor: Rogalla, Frank, 28050 Madrid (ES); Monsalvo Garcia, Victor, 28050 Madrid (ES); Icaran Lopez, Pilar, 28050 Madrid (ES); Fernandez-Polanco, Fernando, 28050 Madrid (ES); Diaz, Israel, 28050 Madrid (ES); Alfaro, Natalia, 28050 Madrid (ES); Fernandez-Polanco, María, 28050 Madrid (ES)
(74) Representative: Carvajal y Urquijo, Isabel

(57) **Abstract**

The invention relates to a method for obtaining methane-enriched biogas for an anaerobic digester (1) that operates at a self-generated pressure, which is greater than the atmospheric pressure, comprising feeding the digester (1) with organic matter for carrying out anaerobic digestion, extracting an enriched biogas stream (13) through a valve (11); and which further comprises the steps of extracting and recirculating organic matter that is being treated in the digester (1) through a recirculation pipe and a pumping device (14), introducing a recirculation stream (6) which contains organic matter in an external liquid gas mixing system (3) and injecting a hydrogen stream (2), and subsequently injecting a liquid stream of hydrogen-saturated organic matter and microbubbles in a gaseous phase into the digester (1)

## Description

### Object of the invention

The present invention relates to a method for obtaining methane-enriched biogas from organic matter, performed in anaerobic digestion reactors, and a facility for carrying out said method.

The present invention is comprised within the technology of wastewater and solid waste treatment; especially sludge treatment.

### Background

Anaerobic digestion processes are widely used both in the sectors of wastewater and solid waste treatment, as well as the bioenergy sector. A common characteristic of all anaerobic systems is that organic matter is transformed into a gaseous mixture of CH₄ (methane) and CO₂, which is known as biogas, by means of microorganisms. Although the normal composition of biogas varies depending on the chemical composition of the organic matter, it is usually 65% CH₄ and 35% CO₂.

In order to expand the potential uses of biogas it is necessary to increase the concentration of CH₄ with the objective of converting biogas into biomethane, with equivalent characteristics to those of natural gas. An analysis of the processes used indicates that there are two ways to address the problem of further increasing the concentration of methane. A first way is to continue with conventional digestion and treat the biogas produced *ex situ*; while a second way consists of modifying the conventional digestion process, provoking *in situ* methane-rich biogas production, and even the production of biomethane.

Within the *ex situ* systems for treating the biogas produced, the most commonly used washing technologies (biogas upgrading) are the physical-chemical methods, in which the traditionally used technologies are: high pressure water washing, amine absorption and pressure swing adsorption (PSA) cycles.

Recently, processes that apply selective membrane technology have been developed and implemented. In the last few years biological processes that are based on the use of hydrogenotrophic microorganisms, which are able to carry out the reaction CO₂ + 4H₂ → CH₄ + 2H₂O with relatively fast kinetic variables, have been studied and developed.

While physical-chemical processes separate CO₂ and release it into the atmosphere, biological processes are able to transform CO₂ into CH₄. With regard to the way *ex situ* biological processes are put into practice, there are two variables that allow the technologies developed to be grouped together. The first is the operating pressure; the second, the way of supplying H₂.

Gang L. and Angelidaki I. ("Integrated biogas upgrading and hydrogen utilization in an anaerobic reactor containing enriched hydrogenotrophic methanogenic culture", Biotechnology and Bioengineering, Vol. 109, Issue 11; November 2012) describe a method for enriching biogas produced in a reactor (*ex situ* process) which is separated from the biodigester. In said separate reactor, hydrogen and a biogas stream produced in the digester are mixed.

Patent application US2014/0342426 "Methods and apparatus for hydrogen based biogas upgrading" describes and claims *ex situ* treatment of biogas in bioreactors at atmospheric pressure into which H₂ is injected.

Patent application WO2014/016815 "Production of methane", describes and claims a method for producing hydrocarbons from CO₂ and H₂ in a system that is pressurized and in which electrodes are introduced in order to achieve the electrolysis of water.

In *in situ* processes, the reaction of transforming CO₂ into CH₄ is done in the digester itself. Just as in the case of *ex situ* processes, there are two variables that allow the technology developed to be grouped together, the first being the operating pressure; the second, the way in which H₂ is supplied.

With regard to utilizing the effect of the pressure on the dissolution of CO₂, scientific literature presents and discusses experimental data on improvements to the concentration of CH₄ in reactors that operate with pressure: the CO₂ is converted to a liquid phase, but does not transform into CH₄ (Lindeboom et al. Water Science and Technology 2011. 64, 647-653; Lindeboom et al. Environmental Science and Technology. 2012. 46, 1895-1902).

Patent application DE102011015415 A1, "Druckmethanisierung von biomasse", uses two-stage digestion wherein the hydrolyzate of the first stage passes to a reactor that operates with pressure.

Patent application WO 2011/005079 A1, "System and method for purifying an aqueous organic matter waste and/or wastewater under pressure and method for producing biogas", is another example of a pressure reactor. In none of the cases is H₂ produced or injected inside the reactor.

Revising the state of the art based on reactors that operate with H₂, patent application US2014/0342426, "Methods and apparatus for hydrogen based biogas upgrading", claims *in situ* treatment of biogas in bioreactors under atmospheric pressure in which H₂ is injected in order to facilitate control of the pH, and acid substrates are introduced in the feeding.

Patent application US2012/0100590 "Microbially assisted water electrolysis for improving biomethane production", also operates under atmospheric pressure, but H₂ is produced electrolytically. Both processes operate under atmospheric pressure.

Patent MD4376B1 (31/10/2015), "Combined high-pressure biogas production reactor", describes a process which operates with a high-pressure reactor and utilizes the reaction of the biological transformation of carbon dioxide into methane; the main design characteristic of this document being a specific and complex anaerobic reactor, clearly specified in claim 1. Furthermore, the reactor is necessarily coupled to an electrolytic system to produce H₂, hence the title "combined reactor".

Considering the aforementioned prior art, the method described below is applied to any type of anaerobic technology for treating both wastewater and solid waste, without requiring a specific reactor.

On the other hand, the invention allows for an H₂ injection from any external source and without the need to couple an electrolytic system. It is well known by a person skilled in the art that the biological process of digestion is limited by the value of the coefficient of the hydrogen transfer to the liquid phase ("A feasibility study on the bioconversion of CO2 and H2 to biomethane by gas sparging through polymeric membranes", Diaz I .et al. Bioresource Technology. Vo. 185, June, 2015, pages 246-253). The value of the kLa coefficient of hydrogen is relatively low and, according to Raoult's law, increases with pressure. Anaerobic digesters operate at atmospheric pressure or at a very moderate excess pressure, around 200 mm w.c (1.96 kPa). Therefore, to improve matter transfer it is necessary for digesters to operate at higher pressures. Furthermore, it is also known that correct maintenance of the inside of anaerobic digesters is very complicated and time consuming, which is why there is a tendency for auxiliary equipment to be placed on the outside of the digesters.

### Description of the invention

A first aspect of the invention relates to a method for obtaining methane-enriched biogas, to be put into practice in an anaerobic digester that operates at a pressure that is self-generated by the digester itself, the value of which is greater than atmospheric pressure. The method comprises feeding the digester with organic matter, which may be a stream of wastewater coming from a factory, or solid organic matter, such as sludge from any water treatment facilities. Once the organic matter is introduced in the digester, it will be subjected to anaerobic digestion. Other operations comprised in this method are the extraction of a methane-enriched biogas stream through a valve located at the outlet of the digester, which is configured to control and maintain the self-generated pressure.

Furthermore, the novel stages of the method with respect to the state of the art are the following: extracting and recirculating organic matter that is being treated or digested in the anaerobic digester, introducing a recirculation stream that contains partially digested organic matter and also injecting a hydrogen flow in an external system of liquid and gas mixing; this external mixing system being configured to control the hydrogen flow and in order for the stream of organic matter to reach the condition of saturation; subsequently, injecting into the digester a stream based on a liquid mixture of hydrogen-saturated organic matter and microbubbles in a gaseous phase coming from the external mixing system.

A person skilled in the art knows that hydrogen transfer from a gaseous phase to liquid phase is the limiting step, and therefore it is necessary to have a mixing system that facilitates fast hydrogen dissolution and allows for the condition of saturation to be reached. In practice, reactors or digesters use different systems to achieve a good mixture and the resulting homogenization of the phase in digestion. The most commonly used technologies are: mechanical stirring with blades that rotate inside, stirring through external recirculation in the digestion phase, stirring through the recirculation of biogas.

According to the way in which the stirring and mixing process is done in the digestion phase, there are different possibilities for injecting hydrogen into the reactor. For high-volume reactors, mechanical stirring systems alone are not able to achieve a fast dissolution of hydrogen. Conventional gas stirring systems are not efficient when working with large bubbles. In both cases, the transfer speed of hydrogen is not high enough.

Considering the foregoing, the mixture of the method of the invention is done outside the digestion process and aims to increase hydrogen transfer to the liquid phase based on the recirculation stream coming from the digester.

In this way, the method of the invention enables methane-enriched biogas to be obtained without the need for an external treatment of the biogas produced. Likewise, the invention is able to introduce the necessary hydrogen in order for the hydrogenotrophic microorganisms to transform carbon dioxide into methane.

Anaerobic digestion is done at a temperature within any of the temperature ranges where psychrophilic, mesophilic, thermophilic and hyperthermophilic microorganisms are present. The temperature at which the method is carried out is comprised between 5 and 95°C.

A second aspect of the invention relates to a facility for obtaining methane-enriched biogas by means of a method defined according to the aforementioned method.

The facility comprises: a digester, configured for anaerobic digestion of organic matter, a pH control system in the digester, an external liquid gas mixing system configured in order for a liquid phase to be saturated with hydrogen, a pump system configured to inject a hydrogen-saturated liquid mixture into the anaerobic digester, and a control device configured to measure the hydrogen and carbon dioxide concentrations present in the biogas obtained from the reactor and regulate the recirculation flow.

According to one embodiment, the control device of the facility is configured to regulate the pressure generated by the gas in the digester between 0.5 barg (0.05 MPa) and 50 barg (5 MPa) in the digester.

According to another embodiment, the external mixing system is configured to generate a hydrogen-saturated liquid flow and microbubbles in a gaseous phase that feeds the digester.

Optionally, the hydrogen-saturated liquid flow is transported to the digester by means of a mixing-centrifugal pump.

According to another option, the hydrogen-saturated liquid flow is injected into the digester by means of a Venturi-type ejector.

The advantages of the invention with respect to the state of the art are:
- the method may be used in anaerobic reactors of any technology, without the need to make modifications or install internal equipment in the digester.
- the facility may be sized independently, without the need to combine digestion and hydrogen injection into a single process.
- the use of external liquid gas mixers, of reduced volume in comparison with the volume of the digester, allows for a fine adjustment of the process and a control that guarantees saturation of H₂ in the liquid flow which is introduced in the digester.
- the external gas liquid mixers enable the hydrogen that remains in a gaseous phase to do so in the form of microbubbles which increases the hydrogen transfer inside the digester.
- the method makes it so the pressure inside the reactor or digester is up to 50 bars (5 MPa) and there is a self-generated pressure in the digester itself due to the production of gas.

### Description of the figures

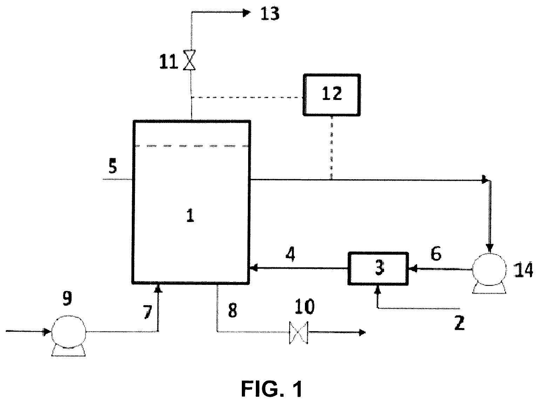

Figure 1 represents a diagram of the method of the invention.

### Exemplary embodiment

An example of the method and facility is described below according to the invention. This description is supported by figure 1.

The anaerobic digestion reactor, or digester (1), is fed a stream (7) of organic matter to be digested, which is pumped by the pump (9). In this example, the facility is fed sludge, which comes from a wastewater treatment plant (not shown).

The outlet of sludge from the digester (1) is done through the piping (8) and the digester may be depressurized by means of the valve (10). The pressure reached (greater than atmospheric pressure) inside the digester is due to the production of methane-rich biogas. This internal pressure is regulated by the valve (11), which operates automatically and allows the enriched biogas (13) produced in the digester to be released or extracted the moment the pre-established pressure (set point) is exceeded. This behavior corresponds to an anaerobic reactor operating with self-generated pressure.

In order for the hydrogenotrophic microorganisms inside of the digester (1) to be able to consume the carbon dioxide by means of the reaction CO₂ + 4H₂ → CH₄ + 2H₂O, it is necessary to introduce the necessary hydrogen. The amount of hydrogen to be introduced may be adjusted based on the biogas composition produced and the stoichiometry of the reaction.

In the present facility, and according to the method of the invention, the hydrogen stream (2) is introduced in the external liquid gas mixng system (3), which in turn receives the recirculation stream (6) driven by the centrifugal pump (14). In this example, the external liquid gas mixing system (3) is made up of a centrifugal pump that is specially designed to mix liquids and gases. The external hydrogen supply source is not shown. Any person skilled in the art knows the different sources of hydrogen.

The stream (4), which is driven by the pump (14) to be introduced in the digester (1), is based on the mixture of the recirculation stream of partially digested organic matter that is practically hydrogen saturated at operational pressure and by hydrogen microbubbles in a gaseous phase.

The stream (4) is injected into the digester (1) by means of a Venturi-type ejector, with the purpose of being mixed with the organic matter being digested present in the digester, which contains dissolved carbon dioxide. In this way, the hydrogenotrophic microorganisms have hydrogen and carbon dioxide in dissolution and can carry out the transformation of organic matter into methane.

By means of a control device (12), the concentrations of hydrogen and carbon dioxide present in the biogas (13) that comes out of the digester (1) are measured and the flow of the recirculation stream (6) of the organic matter being treated is regulated.

## Claims

1. A method for obtaining methane-enriched biogas, to be carried out in an anaerobic digester (1) that operates at a self-generated pressure in the digester itself, which is greater than the atmospheric pressure, comprising feeding the digester (1) with organic matter that is treated by means of anaerobic digestion, and extracting an enriched biogas stream (13) by means of a valve (11) configured to control and maintain the self-generated pressure, **characterized by** the following steps: extracting and recirculating organic matter that is being treated in the digester (1) through a recirculation pipe and a pumping device (14); introducing a recirculation stream (6) of organic matter into an external liquid gas mixing system (3) and injecting a hydrogen flow (2), wherein said external mixing system (3) is configured to control the flow of hydrogen and so that the stream of organic matter reaches the condition of saturation; and, subsequently injecting into the digester (1) a liquid mixture stream (4) of hydrogen-saturated organic matter and microbubbles in a gaseous phase coming from the external mixing system (3).

2. The method according to claim 1, **characterized in that** the anaerobic digestion is carried out at a temperature that is between 5 and 95°C.

3. A facility for obtaining methane-enriched biogas by means of a method defined in claim 1, **characterized in that** it comprises: an anaerobic digester (1), configured for the anaerobic digestion of organic matter, a pH control system (5) in the digester, an external liquid gas mixing system (3) wherein a liquid phase is saturated with hydrogen, a pumping device (14) configured to inject a hydrogen-saturated liquid stream (4) into the digester (1), and a control device (12) configured to measure the hydrogen and carbon dioxide concentrations present in the biogas (13) which exits the digester (1), and to regulate the recirculation flow (6).

4. The facility according to claim 3, **characterized in that** the control device (12) is configured to regulate the pressure generated by the gas between 0.5 and 50 barg in the digester (1).

5. The facility according to claim 3, **characterized in that** the external mixing system (3) is configured to generate a hydrogen-saturated liquid flow and microbubbles in a gaseous phase that feeds the digester (1).

6. The facility according to claims 3 to 5, **characterized in that** the hydrogen-saturated liquid flow is transported to the digester (1) by means of a mixing-centrifugal pump.

7. The facility according to claims 3 to 6, **characterized in that** the hydrogen-saturated liquid flow is injected into the digester (1) by means of a Venturi-type ejector.

8. The facility according to claims 3 to 6, **characterized in that** the hydrogen-saturated liquid flow is injected into the digester (1) by means of a static ejector-mixer.
